(19) Europäisches Patentamt
European Patent Office
Office européen des brevets

(11) Numéro de publication : 0 296 979 B1

# (12) FASCICULE DE BREVET EUROPEEN

(45) Date de publication du fascicule du brevet : 04.12.91 Bulletin 91/49

(51) Int. Cl.[5] : C07D 207/273, A61K 31/40

(21) Numéro de dépôt : 88401587.6

(22) Date de dépôt : 23.06.88

## (54) Dérivés de la 1-benzoyl 2-oxo 5-alcoxy pyrrolidine, leur préparation, leur application comme médicament et les compositions les renfermant.

Le dossier contient des informations techniques présentées postérieurement au dépôt de la demande et ne figurant pas dans le présent fascicule.

(30) Priorité : 26.06.87 IT 2107987

(43) Date de publication de la demande : 28.12.88 Bulletin 88/52

(45) Mention de la délivrance du brevet : 04.12.91 Bulletin 91/49

(84) Etats contractants désignés : AT BE CH DE ES FR GB GR IT LI LU NL SE

(56) Documents cités : EP-A- 0 024 030

(73) Titulaire : ROUSSEL-UCLAF
35, Boulevard des Invalides
F-75007 Paris (FR)

(72) Inventeur : Galliani, Giulio
13, Via Silva
I-1 Monza (MI) (IT)
Inventeur : Barzaghi, Fernando
Via Monteverdi 21
I-20052 Monza - Milan (IT)
Inventeur : Zirotti, Carlo
Via 2, Giugno 12
Arona (NO) (IT)
Inventeur : Toja, Emilio
Via Plezzo 80
Milano (IT)

(74) Mandataire : Tonnellier, Marie-José et al
111, route de Noisy B.P. no 9
F-93230 Romainville (FR)

EP 0 296 979 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux dérivés de la 1-benzoyl 2-oxo 5-alcoxy pyrrolidine, leur procédé de préparation, leur application comme médicaments et les compositions les renfermant.

On connaissait déjà des dérivés de la 1-(p-méthoxy benzoyl) 3 ou 4-hydroxy 2-pyrrolidinone utilisables dans le traitement des insuffisances cérébrales ou pour l'amélioration des capacités intellectuelles (cf EP-A-24030). On vient de découvrir de nouveaux dérivés de la 1-benzoyl 2-pyrrolidinone présentant des propriétés pharmacologiques intéressantes.

L'invention a pour objet les composés de formule (I) ;

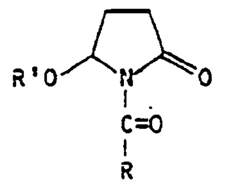

(I)

dans laquelle R' représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2b]-pyranyle, benzoxazolyle ou morpholinyle, le radical R pouvant être éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, le radical éthényle ou le radical éthynyle, les atomes d'halogène, les groupements $CF_3$, $SCF_3$, $OCF_3$, $NO_2$, $NH_2$ ou C≡N, le radical phényle, les groupements acyles et alcoxy-carbonyles renfermant de 2 à 8 atomes de carbone et les groupements alcoylsulfonyles renfermant de 1 à 6 atomes de carbone.

Par radical alcoyle, on entend de préférence un radical renfermant de 1 à 8 atomes de carbone, par exemple, le radical méthyle, éthyle, n-propyle, isopropyle, n-butyle, isobutyle, terbutyle, n-pentyle, n-hexyle, n-heptyle, n-octyle, cyclopropyle, cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

Par un ou plusieurs radicaux alcoyles linéaires, ramifiés ou cycliques, saturés ou insaturés, comportant jusqu'à 18 atomes de carbone, il s'agit par exemple du radical méthyle, éthyle, propyle ou isopropyle, du radical éthényle ou du radical éthynyle.

Lorsque le ou les substituants de R sont des atomes d'halogène, l'halogène concerné est de préférence le fluor, le chlore ou le brome.

L'invention a plus particulièrement pour objet les composés de formule (I) dans lesquels R représente un radical phényle éventuellement substitué par l'un des substituants indiqués ci-dessus ainsi que ceux dans lesquels R représente un radical linéaire, ramifié ou cyclique renfermant jusqu'à 12 atomes de carbone comme par exemple un radical n-pentyle, n-hexyle, n-heptyle ou n-octyle.

L'invention a notamment pour objet les composés de formule (I) dont la préparation est donnée plus loin dans la partie expérimentale.

Parmi les composés préférés de l'invention, on peut citer les composés des exemptes 7 à 10.

Les composés de l'invention présentent d'intéressantes propriétés pharmacologiques : ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicaments, les produits des exemples 7 et 10.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 7 administré par voie orale.

La présente invention a également pour objet les compositions pharmaceutiques renfermant comme principe actif au moins un produit de formule (I).

Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

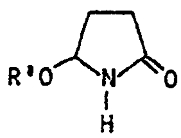

(II)

dans laquelle R' conserve la même signification que précédemment, à l'action d'un composé de formule (III):

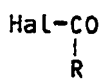

(III)

dans laquelle Hal représente un atome de chlore ou de brome et R conserve la même signification que précédemment, pour obtenir le composé de formule (I) correspondant.

Dans un mode de réalisation préférée du procédé de l'invention, la réaction entre le produit de formule (II) et le produit de formule (III) est effectuée :

a) en présence d'une base forte comme la pyridine, le butyllithium, un hydrure alcalin, comme l'hydrure de sodium, ou le bis-(triméthylsilyl) amidure de sodium ;

b) au sein d'un solvant choisi dans le groupe constitué par le tétrahydrofuranne, le benzène, le diméthylformamide, le diméthylsulfoxyde, le dioxane, ou l'éther diéthylique du diéthylène glycol.

Les produits de formule (II) utilisés comme produits de départ sont des produits connus d'une façon générale qui peuvent être préparés selon le procédé décrit dans Tetrahedron 31, 1437 (1975) ou Tetrahedron 41, 2007 (1985) ou selon le procédé décrit dans Heterocycles 22, 1733 (1984).

La préparation de certains produits de formule (II) est donnée plus loin dans la partie expérimentale.

Les exemples suivants illustrent l'invention, sans toutefois la limiter.

**Exemple 1 : 1-benzoyl 5-éthoxy pyrrolidin-2-one.**

A une solution de 11 g de 5-éthoxy pyrrolidin-2-one dans 64 cm3 de pyridine refroidie à −5°C, on ajoute 12 g de chlorure de benzoyle, agite pendant 8 heures à 0°C. Après 16 heures de repos, on agite à nouveau pendant 8 heures à température ambiante, dilue avec 500 cm3 d'eau et extrait avec du chloroforme. On évapore le solvant et chromatographie le résidu sur silice (éluant : benzène-acétate d'éthyle 5-2) puis évapore le solvant et distille le résidu sous 0,5 mm Hg. On lave le produit obtenu avec une solution aqueuse de bicarbonate de sodium, extrait avec du chloroforme puis évapore le solvant. On obtient 2,1 g de produit attendu. F = 53-55°C isolé de l'éther de pétrole. L'échantillon pour l'analyse a été distillé a 130-135°C sous 0,5 mm Hg.

| | | | | |
|---|---|---|---|---|
| Analyse | : | $C_{13}H_{15}NO_3$ | | |
| Calculé | : | C% 66,68 | H% 6,37 | N% 6,03 |
| Trouvé | : | 66,93 | 6,48 | 6,00 |

**Exemple 2 : 1-(1-méthoxybenzoyl) 5-éthoxy pyrrolidin-2-one.**

A une solution de 7 g de 5-éthoxy pyrrolidin- 2-one dans 150 cm3 de dioxane, on ajoute 2,6 g d'hydrure de sodium (dispersé à 55-60% dans l'huile) et agite 1 heure. A ajoute une solution de 9,25 g de chlorure de

4-méthoxy benzoyle dans 60 cm3 de dioxane et agite pendant 4 heures. On filtre puis évapore le dioxane sous pression réduite. On chromatographie le résidu sur 500 g de gel de silice (éluant : acétone-n-hexane 1-2) et obtient 6,35 g de produit attendu. Eb : 210°C sous 0,09 mbar.

| Analyse : | $C_{14}H_{17}NO_4$ | | |
|---|---|---|---|
| Calculé : | C% 63,86 | H% 6,51 | N% 5,32 |
| Trouvé : | 63,57 | 6,44 | 5,27 |

**Exemple 3 : 1-benzoyl 5-n-propyloxy pyrrolidin-2-one.**

A une solution de 4 g de 5-(1-propyloxy) pyrrolidin-2-one dans 80 cm3 de tétrahydrofuranne à −60°C, on ajoute 18,6 cm3 d'une solution 1,5M de n-butyllithium dans l'hexane en maitenant la température entre −55°C et −60°C. On agite pendant 15 minutes à −60°C puis ajoute à cette température une solution de 3,93 g de chlorure de benzoyle dans 20 cm3 de tétrahydrofuranne. On laisse revenir à température ambiante puis évapore le solvant sous pression réduite. On chromatographie le résidu sur silice (éluant : acétate d'éthyle-n-hexane 1-3) et obtient 4 g de produit attendu. Eb : 200°C sous 0,1 mbar.

| Analyse : | $C_{14}H_{17}NO_3$ | | |
|---|---|---|---|
| Calculé : | C% 68,00 | H% 6,93 | N% 5,66 |
| Trouvé : | 67,77 | 6,82 | 5,74 |

**Exemple 4 : 1-benzoyl 5-isopropyloxy pyrrolidin-2-one.**

A une solution de 2,5 g de 5-isopropyloxy pyrrolidin-2-one dans 60 cm3 de tétrahydrofuranne, an ajoute à −70°C 11,7 cm3 d'une solution 1,6M de n-butyllithium dans l'hexane en maintenant la température entre −70°C et −65°C. On agite pendant 30 minutes dans ces conditions puis ajoute une solution de 2,46 g de chlorure de benzoyle dans 8 cm3 de tétrahydrofuranne en maintenant la température entre −65°C et −70°C. On laisse revenir à température ambiante pendant 2 heures. On évapore à sec puis chromatographie sur silice (éluant : acétate d'éthyle-n-hexane 1-3) et obtient 2,78 g de produit recherché.

| Analyse : | $C_{14}H_{17}NO_3$ | | |
|---|---|---|---|
| Calculé : | C% 68,00 | H% 6,93 | N% 5,66 |
| Trouvé : | 68,12 | 7,04 | 5,74 |

**Exemple 5 : 1-benzoyl 5- butyloxy pyrrolidin-2-one.**

A une solution de 4 g de 5-(1-butyloxy) pyrrolidin-2-one dans 90 cm3 de tétrahydrofuranne, on ajoute à −60°C 17 cm3 d'une solution 1,5M de n-butyllithium dans le n-hexane. On agite pendant 15 minutes à −60°C puis à cette température, on ajoute une solution de 3,58 g de chlorure de benzoyle dans 25 cm3 de tétrahydrofuranne. On laisse revenir à température ambiante, évapore le solvant sous pression réduite et chromatographie le résidu sur silice (éluant : acétate d'éthyle-n-hexane 1-3) et obtient 4 g de produit attendu. Eb : 200-210°C sous 0,07 mbar.

| Analyse : | $C_{15}H_{19}NO_3$ | | |
|---|---|---|---|
| Calculé : | C% 68,94 | H% 7,33 | N% 5,36 |
| Trouvé : | 68,86 | 7,40 | 5,54 |

**Exemple 6 : 1-(1-méthoxybenzoyl) 5-n-butyloxy pyrrolidin-2-one.**

A une solution refroidie à −45°C, de 2,8 g de 5-n-butyloxy pyrrolidin-2-one dans 70 cm3 de tétrahydrofuranne, on ajoute 11,2 cm3 d'une solution 1,6M de n-butyllithium dans le n-hexane à −40°C, agite 30 minutes et dans les mêmes conditions, on ajoute une solution de 5,24 g de chlorure d'anisoyle (à 58% dans le toluène) dans 6 cm3 de tétrahydrofuranne. On agite 2 heures en laissant revenir à température ambiante puis évapore à sec. On chromatographie l'huile obtenue sur silice (éluant : toluène-acétate d'éthyle 8-2) et obtient 2 g de produit recherché.

| Analyse : | $C_{16}H_{21}NO_4$ | | |
|---|---|---|---|
| Calculé : | C% 65,96 | H% 7,26 | N% 4,81 |
| Trouvé : | 65,39 | 7,36 | 5,03 |

**Exemple 7 : 1-benzoyl 5-n-pentyloxy pyrrolidin-2-one.**

A une solution de 4 g de 5-(1-pentyloxy) pyrrolidin-2-one dans 80 cm3 de tétrahydrofuranne, on ajoute à −60°C 15.56 cm3 d'une solution 1,5M de n-butyllithium dans l'hexane. On agite 15 minutes à −60°C puis dans ces conditions, on ajoute une solution de 3,28 g de chlorure de benzoyle dans 20 cm3 de tétrahydrofuranne. On laisse revenir à température ambiante puis évapore le solvant sous pression réduite. On chromatographie le résidu sur silice (éluant : acétate d'éthyle-n-hexane 1-3) et obtient 4 g de produit recherché.

| Analyse : | $C_{16}H_{21}NO_3$ | | |
|---|---|---|---|
| Calculé : | C% 69,79 | H% 7,69 | N% 5,09 |
| Trouvé : | 69,62 | 7,78 | 5,19 |

**Exemple 8 : 1-(4-méthoxybenzoyl) 5-n-pentyloxy pyrrolidin-2-one.**

A une solution de 4 g de 5-(1-pentyloxy) pyrrolidin-2-one dans 80 cm3 de tétrahydrofuranne, on ajoute à −70°C 15,57 cm3 d'une solution 1,5M de n-butyllithium dans le n-hexane. On agite 15 minutes à −70°C puis en maintenant cette température, on ajoute une solution de 3,98 g de chlorure de para-anisoyle à 58% dans le toluène. On laisse revenir à température ambiante, évapore sous pression réduite et chromatographie le résidu sur silice (éluant : n-hexane-acétate d'éthyle 2-1). On obtient 5,1 g de produit recherché.

| Analyse : | $C_{17}H_{23}NO_4$ | | |
|---|---|---|---|
| Calculé : | C% 66,86 | H% 7,59 | N% 4,59 |
| Trouvé : | 67,02 | 7,68 | 4,76 |

**Exemple 9 : 1-benzoyl 5-n-hexyloxy pyrrolidin-2-one.**

A 4,5 g de 5-n-hexyloxy pyrrolidin-2-one en solution dans 130 cm3 de tétrahydrofuranne anhydre, on ajoute à −70°C 16 cm3 d'une solution 1,6M de butyllithium dans l'hexane. On agite 20 minutes à −70°C et ajoute dans ces conditions une solution de 3,4 g de chlorure de benzoyle dans 20 cm3 de tétrahydrofuranne. On laisse revenir à température ambiante, concentre sous pression réduite et chromatographie le résidu sur silice (éluant : toluène-acétate d'éthyle 8-2). On obtient 3,3 g de produit recherché.

| Analyse : | $C_{17}H_{23}NO_3$ | | |
|---|---|---|---|
| Calculé : | C% 70,56 | H% 8,01 | N% 4,89 |
| Trouvé : | 70,26 | 7,84 | 4,88 |

**Exemple 10 : 1-benzoyl 5-n-heptyloxy pyrrolidin-2-one.**

A 4,98 g de 5-n-heptyloxy pyrrolidin-2-one en solution dans 100 cm3 de tétrahydrofuranne, on ajoute à −60°C 16,5 cm3 d'une solution de butyllithium à 15% dans l'hexane. Après 20 minutes d'agitation à −60°C,

on ajoute à cette température 3,5 g de chlorure de benzoyle dans 25 cm3 de tétrahydrofuranne. On agite pendant 4 heures en laissant revenir à température ambiant. On concentre à 40°C, reprend le résidu à l'eau, extrait à l'éther, concentre à sec. On chromatographie le résidu sur silice (éluant : toluène-acétate d'éthyle 8-2) et obtient 1,2 g de produit recherché.

| Analyse : | $C_{18}H_{25}NO_3$ | | |
|---|---|---|---|
| Calculé : | C% 71,26 | H% 8,31 | N% 4,62 |
| Trouvé : | 71,54 | 8,25 | 4,53 |

**Exemple 11 : 1-benzoyl 5-n-octyloxy pyrrolidin-2-one.**

A 5,3 g de 5-n-octyloxy pyrrolidin-2-one en solution dans 200 cm3 de tétrahydrofuranne, on ajoute à –60°C 16,5 cm3 d'une solution de butyllithium à 15% dans l'hexane. On agite pendant 20 minutes à –60°C puis en maintenant ces conditions, on ajoute une solution de 3,5 g de chlorure de benzoyle dans 50 cm3 de tétrahydrofuranne. On agite en laissant revenir à température ambiante. On concentre et chromatographie sur silice (éluant : toluène-acétate d'éthyle 8-2). On obtient 4,2 g de produit recherché.

| Analyse : | $C_{19}H_{27}NO_3$ | | |
|---|---|---|---|
| Calculé : | C% 71,89 | H% 8,57 | N% 4,41 |
| Trouvé : | 71,69 | 8,48 | 4,32 |

**Exemple 12 : 1-benzoyl 5-méthoxy pyrrolidin-2-one.**

A une solution de 3,8 g de 5-méthoxy pyrrolidin-2-one dans 160 cm3 de tétrahydrofuranne, on ajoute à –70°C 20,8 cm3 d'une solution 1,5M de butyllithium dans l'hexane. On maintient cette température pendant 20 minutes puis ajoute 4,69 g de chlorure de benzoyle en solution dans le tétrahydrofuranne, laisse revenir à température ambiante, concentre à sec et chromatographie sur silice (éluant : hexane-acétate d'éthyle 1-1). On obtient 3 g de produit attendu. F = 92-94°C cristallisé dans l'éther isopropylique.

| Analyse : | $C_{12}H_{13}NO_3$ | | |
|---|---|---|---|
| Calculé : | C% 65,74 | H% 5,98 | N% 6,39 |
| Trouvé : | 65,92 | 5,96 | 6,32 |

**Exemple 13 : 1-(3-trifluorométhyl) benzoyl 5-n-pentyloxy pyrrolidin-2-one.**

A une solution de 4,5 g de 5-n-pentyloxy pyrrolidin-2-one dans 160 cm3 de tétrahydrofuranne, on ajoute à –70°C 17,5 cm3 d'une solution 1,5M de butyllithium dans l'hexane. On maintient cette température pendant 20 minutes puis ajoute 5,48 g de chlorure de 3-trifluorométhyl benzoyle en solution dans 20 cm3 de tétrahydrofuranne. On laisse revenir à température ambiante, concentre à sec et chromatographie sur silice (éluant: hexane-acétate d'éthyle 75-25). On obtient 6,1 g de produit attendu.

| Analyse : | $C_{17}H_{20}F_3NO_3$ | | |
|---|---|---|---|
| Calculé : | C% 59,47 | H% 5,87 | N% 4,08 |
| Trouvé : | 59,39 | 5,81 | 4,17 |

**Exemple 14 : 1-(4-nitro) benzoyl 5-n-pentyloxy pyrrolidin-2-one.**

A 4 g de 5-pentyloxy pyrrolidin-2-one en solution dans 150 cm3 de tétrahydrofuranne, on ajoute à –70°C 15,5 cm3 d'une solution 1,5M de butyllithium dans l'hexane. On maintient pendant 20 minutes à cette température puis ajoute une solution de 4,33 g de chlorure de 4-nitrobenzoyle dans 20 cm3 de tétrahydrofuranne.

On laisse revenir à température ambiante, concentre, chromatographie sur silice (éluant : hexane-acétate d'éthyle 8-2) et obtient, après cristallisation dans l'isopropanol, 3,9 g de produit. F = 56-58°C,

| | | | |
|---|---|---|---|
| Analyse : $C_{16}H_{20}N_2O_5$ | | | |
| Calculé : | C% 59,99 | H% 6,29 | N% 8,74 |
| Trouvé : | 60,17 | 6,16 | 8,67 |

**Exemple 15 : 1-(4-biphényl) carbonyl 5-n-pentyloxy pyrrolidin-2-one.**

A un mélange de 3,95 g de 5-n-pentyloxy pyrrolidin-2-one dans 150 cm3 de tétrahydrofuranne, on ajoute à −70°C 15,3 cm3 d'une solution 1,5M de butyllithium dans l'hexane. On maintient pendant 20 minutes à cette température puis ajoute une solution de chlorure de 4-biphénylyl carbonyle dans 20 cm3 de tétrahydrofuranne. On laisse revenir à température ambiante, concentre à sec et chromatographie sur silice (éluant : hexane-acétate d'éthyle 8-2). On obtient, après cristallisation dans l'isopropanol, 4,9 g de produit attendu. F = 102-104°C.

| | | | |
|---|---|---|---|
| Analyse : $C_{22}H_{25}NO_3$ | | | |
| Calculé : | C% 75,18 | H% 7,17 | N% 3,99 |
| Trouvé : | 75,02 | 7,15 | 4,14 |

**Exemple 16 : 1-(4-fluoro) benzoyl 5-n-pentyloxy pyrrolidin-2-one.**

A une solution de 2,3 g de 5-n-pentyloxy pyrrolidin-2-one dans 110 cm3 de tétrahydrofuranne, on ajoute à −70°C 8,95 cm3 d'une solution 1,5M de butyllithium dans l'hexane. On maintient 20 minutes à cette température puis ajoute une solution de 2,1 g de chlorure de 4-fluorobenzoyle dans 10 cm3 de tétrahydrofuranne. On ramène à température ambiante, concentre à sec et chromatographie sur silice (éluant hexane-acétate d'éthyle 7-3). On obtient 1,6 g de produit attendu.

| | | | |
|---|---|---|---|
| Analyse : $C_{16}H_{20}FNO_3$ | | | |
| Calculé : | C% 65,51 | H% 6,87 | N% 4,77 |
| Trouvé : | 65,87 | 6,91 | 4,69 |

**Exemple 17 : 1-(4-chloro) benzoyl 5-n-pentyloxy pyrrolidin-2-one.**

A une solution de 2,3 g de 5-n-pentyloxy pyrrolidin-2-one dans 130 cm3 de tétrahydrofuranne, on ajoute à −70°C 8,4 cm3 d'une solution 1,6M de butyllithium dans l'hexane. On maintient pendant 20 minutes à cette température et ajoute une solution de 2,35 g de chlorure de 4-chlorobenzoyle dans 10 cm3 de tétrahydrofuranne. On ramène à température ambiante, concentre à sec et chromatographie sur silice (éluant : hexane-acétate d'éthyle 7-3). On obtient 1,9 g de produit attendu.

| | | | |
|---|---|---|---|
| Analyse : $C_{16}H_{20}ClNO_3$ | | | |
| Calculé : | C% 62,03 | H% 6,51 | N% 4,52 |
| Trouvé : | 62,34 | 6,66 | 4,68 |

**Exemple 18 : 1-benzoyl 5-(3-méthylbutoxy) pyrrolidin-2-one.**

A une solution de 3,3 g de 5-(3-méthylbutoxy) pyrrolidin-2-one dans 140 cm3 de tétrahydrofuranne, on ajoute à −70°C 12,8 cm3 d'une solution 1,5M de butyllithium dans l'hexane. On maintient 20 minutes à cette température puis ajoute une solution de 2,7 g de chlorure de benzoyle dans 20 cm3 de tétrahydrofuranne. On ramène la solution a température ambiante. On concentre à sec sous pression réduite et chromatographie sur silice (éluant : hexane-acétate d'éthyle 7-3). On obtient 3,5 g de produit attendu.

| | | | |
|---|---|---|---|
| Analyse : | $C_{16}H_{21}NO_3$ | | |
| Calculé : | C% 69,79 | H% 7,69 | N% 5,09 |
| Trouvé : | 69,78 | 7,69 | 5,08 |

**Exemple 19 : 1-benzoyl 5-(2-pentyloxy) pyrrolidin-2-one.**

A une solution de 3,2 g de 5-(2-pentyloxy) pyrrolidin-2-one dans 130 cm3 de tétrahydrofuranne, on ajoute à −70°C 12,4 cm3 d'une solution 1,5M de butyllithium dans l'hexane. On maintient 20 minutes à cette température puis ajoute une solution de 2,62 g de chlorure de benzoyle dans 20 cm3 de tétrahydrofuranne. On ramène a température ambiante, concentre et chromatographie sur silice (éluant : hexane-acétate d'éthyle 7-3). On obtient 3,35 g de produit attendu.

| | | | |
|---|---|---|---|
| Analyse : | $C_{16}H_{21}NO_3$ | | |
| Calculé : | C% 69,79 | H% 7,69 | N% 5,09 |
| Trouvé : | 69,91 | 7,62 | 5,07 |

**Exemple 20 : 1-benzoyl 5-n-néopentyloxy pyrrolidin-2-one.**

A une solution de 2,6 g de sodium bis triméthylsilylamide dans 160 cm3 d'éther éthylique, on ajoute à température ambiante 2,2 g de 5-néopentyloxy pyrrolidin-2-one dans 30 cm3 d'éther éthylique. Après 30 minutes, on refroidit la solution à 0°C et ajoute 1,89 g de chlorure de benzoyle dilué avec 20 cm3 d'éther éthylique. On laisse 30 minutes à 0°C puis laisse revenir à température ambiante. On concentre à sec sous pression réduite. On chromatographie sur silice (éluant : hexane-acétate d'éthyle 7-3) et obtient, après cristallisation dans l'éthanol, 1,45 g de produit attendu. F = 83-85°C.

| | | | |
|---|---|---|---|
| Analyse : | $C_{16}H_{21}NO_3$ | | |
| Calculé : | C% 69,79 | H% 7,69 | N% 5,09 |
| Trouvé : | 69,63 | 7,55 | 5,13 |

**Exemple 21 : 1-benzoyl 5-cyclopentyloxy pyrrolidin-2-one.**

A une solution de 3 g de 5-cyclopentyloxy pyrrolidin-2-one dans 140 cm3 de tétrahydrofuranne, on ajoute à −70°C 11,8 cm3 d'une solution 1,5M de butyllithium dans l'hexane. On maintient 20 minutes à cette température puis ajoute une solution de 2,49 g de chlorure de benzoyle dans 20 cm3 de tétrahydrofuranne. On réchauffe lentement puis amène à sec sous pression réduite. On chromatographie sur silice (éluant : hexane-acétate d'éthyle 7-3) et obtient 3,4 g de produit attendu.

| | | | |
|---|---|---|---|
| Analyse : | $C_{16}H_{19}NO_3$ | | |
| Calculé : | C% 70,31 | H% 7,01 | N% 5,12 |
| Trouvé : | 70,06 | 6,93 | 5,15 |

**Exemple 22 : 1-(4-nitro) benzoyl 5-éthoxy pyrrolidin-2-one.**

A une solution de 2,5 g de 5-éthoxy pyrrolidin-2-one dans 110 cm3 de tétrahydrofuranne, on ajoute à −70°C 12,9 cm3 d'une solution 1,5M de butyllithium dans l'hexane. Après 20 minutes, on ajoute à la même température 3,6 g de chlorure de 4-nitrobenzoyle en solution dans du tétrahydrofuranne. On ramène à température ambiante puis concentre à sec sous pression réduite. Après cristallisation dans l'éthanol, on obtient 2,5 g de produit attendu. F = 102-104°C.

| | | | |
|---|---|---|---|
| Analyse : | $C_{13}H_{14}N_2O_5$ | | |
| Calculé : | C% 56,11 | H% 5,07 | N% 10,07 |
| Trouvé : | 56,15 | 5,01 | 9,96 |

**Exemple 23 : 1-(4-biphénylyl) carbonyl 5-éthoxy pyrrolidin-2-one.**

A une solution de 1,5 g de 5-éthoxy pyrrolidin-2-one dans 75 cm3 de tétrahydrofuranne, on ajoute à –70°C 7,7 cm3 d'une solution 1,5M de butyllithium dans l'hexane. Après 20 minutes, on ajoute à cette température 2,5 g de chlorure de 4-biphénylyl carbonyle en solution dans 15 cm3 de tétrahydrofuranne. On ramène à température ambiante et concentre à sec sous pression réduite. On chromatographie sur silice (éluant : hexane-acétate d'éthyle 7-3) et obtient 2,1 g de produit attendu. F = 91-93°C après cristallisation dans l'éthanol.

| | | | |
|---|---|---|---|
| Analyse : | $C_{19}H_{19}NO_3$ | | |
| Calculé : | C% 73,77 | H% 6,19 | N% 4,53 |
| Trouvé : | 73,56 | 6,13 | 4,45 |

**Exemple 24 : 1-(3-trifluorméthyl) benzoyl 5-éthoxy pyrrolidin-2-one.**

A une solution de 2,5 g de 5-éthoxy pyrrolidin-2-one dans 110 cm3 de tétrahydrofuranne, on ajoute à –70°C 12,9 cm3 d'une solution 1,5M de butyllithium dans l'hexane. Après 20 minutes à cette température, on ajoute une solution de 4,04 g de chlorure de 3-trifluorométhyl benzoyle dans le tétrahydrofuranne. On réchauffe à température ambiante, concentre et chromatographie sur silice (éluant : hexane-acétate d'éthyle 7-3). On obtient 3,4 g de produit attendu.

| | | | |
|---|---|---|---|
| Analyse : | $C_{14}H_{14}FNO_3$ | | |
| Calculé : | C% 55,82 | H% 4,68 | N% 4,65 |
| Trouvé : | 55,06 | 4,73 | 4,81 |

**Exemple 25 : 1-(1-nitro) benzoyl 5-n-butoxy pyrrolidin-2-one.**

A une solution de 2,4 g de 5-n-butyloxy pyrrolidin-2-one dans 100 cm3 de tétrahydrofuranne, on ajoute à –70°C 10,2 cm3 de butyllithium en solution 1,5M dans l'hexane. Après 20 minutes à cette température, on ajoute une solution de chlorure de 4-nitrobenzoyle dans 15 cm3 de tétrahydrofuranne. On ramène à température ambiante, concentre, reprend à l'eau, filtre et obtient, après cristallisation dans l'éthanol, 2 g de produit attendu. F = 56-59°C.

| | | | |
|---|---|---|---|
| Analyse : | $C_{15}H_{18}N_2O_5$ | | |
| Calculé : | C% 58,82 | H% 5,92 | N% 9,15 |
| Trouvé : | 59,01 | 5,96 | 8,97 |

**Exemple 26 : 1-(1-biphénylyl) carbonyl 5-n-butoxy pyrrolidin-2-one.**

A une solution de 1,8 g de 5-n-butoxy pyrrolidin-2-one dans 85 cm3 de tétrahydrofuranne, on ajoute à –70°C 7,7 cm3 d'une solution 1,5M de butyllithium dans l'hexane. Après 20 minutes à cette température, on ajoute une solution de 2,5 g de chlorure de 4-biphénynyl carbonyle dans le tétrahydrofuranne. On laisse la température remonter puis concentre à sec. On chromatographie sur silice (éluant : hexane-acétate d'éthyle 7-3) et obtient, après cristallisation dans l'isopropanol, 2,5 g de produit attendu. F = 101-103°C.

| | | | |
|---|---|---|---|
| Analyse : | $C_{21}H_{23}NO_3$ | | |
| Calculé : | C% 74,75 | H% 6,87 | N% 4,15 |
| Trouvé : | 74,81 | 6,92 | 4,06 |

**Exemple 27 : 1-(3-trifluorométhyl) benzoyl 5-n-butoxy pyrrolidin-2-one.**

A une solution de 2,3 g de 5-butoxy pyrrolidin-2-one dans 110 cm3 de tétrahydrofuranne, on ajoute à −70°C 9,7 cm3 d'une solution 1,5M de butyllithium dans l'hexane. Après 20 minutes à cette température, on ajoute une solution de 3,06 g de chlorure de 3-trifluorométhyl benzoyle dans le tétrahydrofuranne. On ramène à température ambiante, concentre à sec et chromatographie sur silice (éluant : hexane-acétate d'éthyle 7-3). On obtient 3 g de produit attendu.

| | | | |
|---|---|---|---|
| Analyse : | $C_{16}H_{18}F_3NO_3$ | | |
| Calculé : | C% 58,36 | H% 5,51 | N% 4,25 |
| Trouvé : | 58,69 | 5,39 | 4,48 |

**Préparation 1 : 5-isopropoxy pyrrolidin-2-one.**

On refroidit à −10°C 28,4 g de succinimide dans 1200 cm3 d'isopropanol. On ajoute 32,8 g d'hydrure de bore et de sodium, agite pendant 4 heures à 0°C, −10°C et ajoute une solution 2N d'acide chlorhydrique dans l'isopropanol à 0°C en régulant le pH à 2-3. On maintient pendant 2 heures à 0°C, puis neutralise avec une solution isopropanolique de potasse. On évapore le solvant sous pression réduite, extrait au chloroforme et concentre à sec sous pression réduite. On obtient 20,5 g de produit recherché. F = 68-71°C.

**Préparation 2 : 5-n-propyloxy pyrrolidin-2-one.**

On opère de la même manière que pour la préparation 1 en utilisant du n-propanol au lieu de l'isopropanol. On obtient 27,5 g de produit attendu. F = 52-54°C.

**Préparation 3 : 5-n-butyloxy pyrrolidin-2-one.**

On opère de la même manière que pour la préparation 1 en employant le n- butanol. On obtient 7,5 g de produit attendu. F = 36-38°C.

Ce produit peut aussi être obtenu par alcoxylation anodique de la pyrrolidin-2-one selon le procédé décrit dans Synthesis 4, 315-7 1980).

**Préparation 4 : 5-n-pentyloxy pyrrolidin-2-one.**

On agite à 65°C pendant 3 heures un mélange de 2,5 g de 5-hydroxy pyrrolidine-2-one et 1,25 g de résine Amberlite® IR 120 H dans 55 cm3 de n-pentanol. On refroidit à température ambiante, filtre, distille sous pression réduite puis chromatographie le résidu sur silice (éluant : acétate d'éthyle). On obtient 2,68 g de produit recherché. F = 42-43°C.

| | | | |
|---|---|---|---|
| Analyse : | $C_9H_{17}NO_2$ | | |
| Calculé : | C% 63,13 | H% 10,01 | N% 8,18 |
| Trouvé : | 63,31 | 9,95 | 8,27 |

**Préparation 5 : 5-n-hexyloxy pyrrolidin-2-one.**

On agite pendant 3 heures à 60°C un mélange de 0,4 g de 5-hydroxy pyrrolidin-2-one, 10 cm3 de n-hexanol et 0,2 g d'Amberlite® IR 120 H. On laisse refroidir, distille le solvant et chromatographie le résidu sur silice (éluant : acétate d'éthyle). On obtient 0,5 g de produit recherché. F = 35-37°C cristallisé de l'hexane.

| | | | | |
|---|---|---|---|---|
| Analyse : | $C_{10}H_{19}NO_2$ | | | |
| Calculé : | C% 64,83 | H% 10,34 | N% 7,56 |
| Trouvé : | 64,67 | 10,25 | 7,49 |

**Préparation 6 : 5-n-heptyloxy pyrrolidin-2-one.**

On agite à 60°C pendant 4 heures un mélange de 8 g de 5-hydroxy pyrrolidin-2-one, 100 cm3 de n-heptanol et 4 g d'Amberlite® IR 120 H. On filtre, distille à sec et obtient 11,9 g de produit attendu. F = 52-54°C cristallisé de l'hexane.

| | | | |
|---|---|---|---|
| Analyse : | $C_{11}H_{21}NO_2$ | | |
| Calculé : | C% 66,29 | H% 10,62 | N% 7,03 |
| Trouvé : | 66,13 | 10,51 | 6,98 |

**Préparation 7 : 5-n-octyloxy pyrrolidin-2-one.**

On agite pendant 4 heures à 60°C un mélange de 7,5 g de 5-hydroxy pyrrolidin-2-one dans 10 cm3 de n-octanol et 4 g d'Amberlite® IR 120 H. On filtre, concentre à sec et obtient 5,5 g de produit attendu. F = 36-38°C cristallisé de l'hexane. Par recristallisation dans l'hexane F = 38-40°C

| | | | |
|---|---|---|---|
| Analyse : | $C_{12}H_{23}NO_2$ | | |
| Calculé : | C% 67,56 | H% 10,87 | N% 6,57 |
| Trouvé : | 67,32 | 10,73 | 6,69 |

**Préparation 8 : 5-(3-méthylbutoxy) pyrrolidin-2-one.**

On agite pendant 5 heures à température ambiante 5,5 g de 5-éthoxy pyrrolidin-2-one, 30 cm3 de 3-méthyl butanol et 2,75 g de résine Amberlite®-15. On laisse 18 heures au freezer. On obtient après filtration 4,1 g de produit attendu. F = 73-75°C.

| | | | |
|---|---|---|---|
| Analyse : | $C_9H_{17}NO_2$ | | |
| Calculé : | C% 63,13 | H% 10,0 | N% 8,18 |
| Trouvé : | 62,88 | 10,06 | 8,32 |

**Préparation 9 : 5-(2-pentyloxy) pyrrolidin-2-one.**

On agite pendant 5 heures à température ambiante 5,5 g de 5-éthoxy pyrrolidin-2-one, 30 cm3 de 2-pentanol à 2,75 g de résine Amberlite®-15. On filtre, distille sous 1 mm de Hg à 40°C le pentanol en excès. On chromatographie le résidu sur silice (éluant : acétate d'éthyle) et obtient 3,7 g de produit attendu.

**Préparation 10 : 5-néopentyloxy pyrrolidin-2-one.**

On agite à 40°C pendant 2 heures 15 minutes 35 g de 2,2-diméthyl propanol (préalablement fondu à 45°C), 6 g de 5-éthoxy pyrrolidin-2-one et 3 g de résine Amberlite®-15. On filtre et distille le 2,2-diméthyl propanol sous 0,8 mm/Hg à 35°C, le résidu est dissous dans l'hexane, glacé pendant 2 heures. Onobtient 3,2 g de produit attendu. F = 67-69°C cristallisé de l'hexane.

Analyse : $C_9H_{17}NO_2$

Calculé : C% 63,13 H% 10,0 N% 8,18

Trouvé : 62,85 9,81 8,20

**Exemples de comositions pharmaceutiques.**

a) On a préparé des comprimés répondant à la formule suivante :

- Produit de l'exemple 7 .................................... 100 mg
- Excipient q.s. pour un comprimé terminé à .................... 300 mg

(Détail de l'excipient : lactose, amidon de blé, amidon traité, amidon de riz, stéarate de magnésium, talc).

b) On a préparé des gélules répondant à la formule suivante :

- Produit de l'exemple 10 ..................................... 200 mg
- Excipient q.s. pour une gélule terminée à .................... 300 mg

(Détail de l'excipient : talc, stéarate de magnésium, Aérosil®).

## ETUDE PHARMACOLOGIQUE

### Toxicité aiguë et comportement.

Nous avons utilisé des souris mâles ($CD_1$ Charles Rivers) d'un poids de 22-23 g à jeun depuis 16 heures. Les produits leur furent administrés par voie orale aux doses de 1000-500-250 mg/kg.

L'effet des produits sur le comportement des animaux fut évalué suivant la méthode décrite par Irvin (Psychopharmacologia (1968), 13, 222-257) durant les 8 premières heures et à la 24ème heure.

La mortalité fut relevée pendant les 7 jours suivant le traitement.

La $DL_{50}$ a ainsi été trouvée supérieure à 1000 mg/kg pour les produits des exemples 1 à 11.

### Apprentissage et mémorisation.

trous avons utilisé des souris mâles ($CD_1$ Charles Rivers) d'un poids de 25-30 g. Les animaux sont placés dans la partie lumineuse d'un box à deux compartiments communicants par une ouverture (G. Galliani, F. Barzaghi et R. Cesana, Med. Sci. Res. 15, 313-314 (1987)).

A l'instant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale à différentes doses.

Nous avons utilisé de 20 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence avec une courbe dose-réponse en forme de cloche.

Les résultats sont exprimés en pourcentages d'augmentation du temps de latence par rapport aux témoins correspondants. Des résultats obtenus avec 2 produits de référence sont fournis.

Les résultats sont les suivants :

**Pourcentage d'augmentation du temps de latence par rapport aux témoins**

| Produit de l'exemple | Dose mg/kg os | | | | |
|---|---|---|---|---|---|
| | 400 | 200 | 100 | 50 | 25 |
| 7 | - | 55* | 118* | 83* | 32 |
| 10 | 6 | 99* | 66* | 28 | 34 |
| PIRACETAM | - | 20 | 48* | 10 | 19 |
| ANIRACETAM | - | 32 | 88* | 77* | 39 |

* Valeurs statistiquement différentes par rapport aux témoins.

**Conclusion :**

Les produits des exemples 7 et 10 se sont révélés plus actifs que les témoins. Ils améliorent notablement le comportement des animaux dans une gamme de doses plus large que dans le cas de l'Aniracetam ou du Piracetam.

**Revendications**

**Revendications pour les Etats contractants suivants : AT, BE, CH, DE, FR, GB, IT, LI, LU, NL, SE**

1. Les composés de formule (I) :

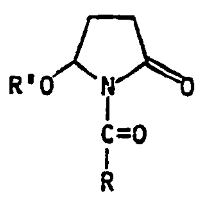

(I)

dans laquelle R' représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2b]-pyranyle, benzoxazolyle ou morpholinyle, le radical R pouvant être éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, le radical éthényle ou le radical éthynyle, les atomes d'halogène, les groupements $CF_3$, $SCF_3$, $OCF_3$, $NO_2$, $NH_2$ ou C≡N, le radical phényle, les groupements acyles et alcoxy-carbonyles renfermant de 2 à 8 atomes de carbone et les groupements alcoylsulfonyles renfermant de 1 à 6 atomes de carbone.

2. Les composés de formule (I) tels que définis à la revendication 1, dans lesquels R représente un radical

phényle éventuellement substitué par l'un des substituants mentionnés dans la revendication 1.

3. Les composés de formule (I) tels que définis à la revendication 1 ou 2, dans lesquels R′ représente un radical alcoyle linéaire, ramifié ou cyclique, renfermant jusqu'à 12 atomes de carbone.

4. Les composés tels que définis à la revendication 3, dans lesquels R′ représente un radical n-pentyle, n-hexyle, n-heptyle ou n-octyle.

5. Les composés de formule (I) tels que définis à la revendication 1, dont les noms suivent :

— la 1-benzoyl 5-(n-pentyloxy) pyrrolidine 2-one,

— la 1-benzoyl 5-(n-heptyloxy) pyrrolidine 2-one.

6. A titre de médicaments, les composés tels que définis à l'une quelconque des revendications 1 à 4.

7. A titre de médicaments, les composés définis à la revendication 5.

8. Les compositions pharmaceutiques renfermant comme principe actif au moins un médicament défini à la revendication 6 ou 7.

9. Procédé de préparation des composés de formule (I) tels que définis à l'une quelconque des revendications 1 à 5, caractérisé en ce que l'on soumet un composé de formule (II) :

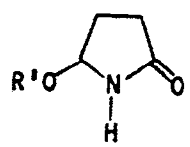

(II)

dans laquelle R′ conserve la même signification que précédemment, à l'action d'un composé de formule (III):

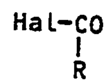

(III)

dans laquelle Hal représente un atome de chlore ou de brome et R conserve la même signification que précédemment, pour obtenir le composé de formule (I) correspondant.

**Revendications pour l'Etat contractant suivant : ES**

1. Procédé de préparation des composés de formule (I) :

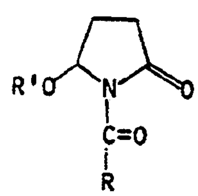

(I)

dans laquelle R′ représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, le radical R pouvant être éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturée ou insaturés, comportant jusqu'à 18 atomes de carbone, le radical éthényle ou le radical éthynyle, les atomes d'halogène, les groupements $CF_3$, $SCF_3$, $OCF_3$, $NO_2$, $NH_2$ ou C≡N, le radical phényle, les groupements acyles et alcoxy-carbonyles renfermant de 2 à 8 atomes de carbone et les groupements alcoyl sulfonyles renfermant de 1 à 6 atomes de carbone, caractérisé en ce que l'on soumet un composé de formule (II)

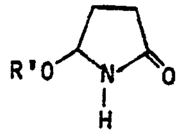

(II)

dans laquelle R′ conserve la même signification que précédemment, à l'action d'un composé de formule (III):

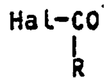

(III)

dans laquelle Hal représente un atome de chlore ou de brome et R conserve la même signification que précédemment, pour obtenir le composé de formule (I) correspondant.

2. Procédé selon la revendication 1, caractérisé en ce que l'on utilise au départ un composé de formule (III) dans laquelle R représente un radical phényle éventuellement substitué par l'un des substituants indiqués à la revendication 1.

3. Procédé selon la revendication 1 ou 2, caractérisé en ce que l'on utilise au départ un composé de formule (II) dans laquelle R′ représente un radical alcoyle linéaire, ramifié ou cyclique, renfermant jusqu'a 12 atomes de carbone.

4. Procédé selon la revendication 3, caractérisé en ce que l'on utilise au départ un composé de formule (II) dans laquelle R′ représente un radical n-pentyle, n-hexyle, n-heptyle ou n-octyle.

5. Procédé selon l'une quelconque des revendications 1 a 4, caractérisé en ce que l'on utilise au départ un composé de formule (II) dans laquelle R′ représente un radical n-pentyle ou n-heptyle et un composé de formule (III) dans laquelle R représente un radical phényle.

**Revendications pour l'Etat contractant suivant : GR**

1. Procédé de préparation des composés de formule (I) :

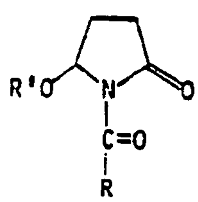

(I)

dans laquelle R′ représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2b]-pyranyle, benzoxazolyle ou morpholinyle, le radical R pouvant être éventuellement substitué par un ou plusieurs substituants choisis dans le groupe constitué par le radical hydroxy, le radical acétoxy, le radical méthoxy ou le radical benzyloxy, les radicaux alcoyles linéaires, ramifiés ou cycliques, saturés ou insaturés, comportant jusqu'à 18 atomes de carbone, le radical éthényle ou le radical éthynyle, les atomes d'halogène, les groupements $CF_3$, $SCF_3$, $OCF_3$, $NO_2$, $NH_2$ ou C≡N, le radical phényle, les groupements acyles et alcoxy-carbonyles renfermant de 2 à 8 atomes de carbone et les groupements alcoyl sulfonyles renfermant de 1 à 6 atomes de carbone, caractérisé en ce que l'on soumet un composé de formule (II)

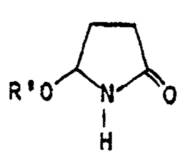

(II)

dans laquelle R′ conserve la même signification que précédemment, à l'action d'un composé de formule (III):

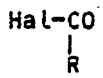

(III)

dans laquelle Hal représente un atome de chlore ou de brome et R conserve la même signification que précédemment, pour obtenir le composé de formule (I) correspondant.

2. Procédé selon la revendication 1, caractérisé en ce que l'on utilise au départ un composé de formule (III) dans laquelle R représente un radical phényle éventuellement substitué par l'un des substituants indiqués à la revendication 1.

3. Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 12 atomes de carbone.

4. Procédé selon la revendication 3, caractérisé en ce que l'on utilise au départ un composé de formule (II) dans laquelle R' représente un radical n-pentyle, n-hexyle, n-heptyle ou n-octyle.

5. Procédé selon l'une quelconque des revendications 1 à 4, caractérisé en ce que l'on utilise au départ un composé de formule (II) dans laquelle R' représente un radical n-pentyle ou n-heptyle et un composé de formule (III) dans laquelle R représente un radical phényle.

6. Procédé de préparation de compositions pharmaceutiques, caractérisé en ce que l'on met, à titre de principe actif, l'un au moins des composés de formule (I), telle que définie à la revendication 1, sous une forme destinée à cet usage.

7. Procédé de préparation de compositions pharmaceutiques, caractérisé en ce que l'on met, à titre de principe actif, l'un au moins des composés de formule (I), telle que définie à l'une quelconque des revendications 2 à 4, sous une forme destinée à cet usage.

8. Procédé de préparation de compositions pharmaceutiques, caractérisé en ce que l'on met, à titre de principe actif, la 1-benzoyl 5-(n-pentyloxy) pyrrolidin-2-one ou la 1-benzyl 5-(n-heptyloxy) pyrrolidin-2-one, sous une forme destinée à cet usage.

## Patentansprüche

### Patentansprüche für folgende vertragsstaaten : AT, BE, CH, DE, FR, GB, IT, LI, LU, NL, SE

1. Verbindungen der Formel (I)

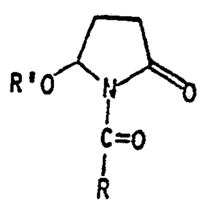

(I)

worin R' für ein Wasserstoffatom, einen linearen, verzweigten oder cyclischen Alkylrest mit bis zu 12 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl oder Biphenylylrest oder einen Furyl-, Thienyl-, Pyranyl-, Pyridyl-, Benzofuranyl-Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno-[2,3-b]-furanyl-, 2H-Furo-[3,2b]-pyranyl-, Benzoxazolyl- oder Morpholinylrest bedeutet, wobei der Rest R gegebenenfalls substituiert sein kann durch einen oder mehrere Substituenten, ausgewählt unter dem Hydroxyrest, dem Acetoxyrest, dem Methoxyrest oder dem Benzyloxyrest, den gesättigten oder ungesättigten linearen, verzweigten oder cyclischen Alkylresten mit bis zu 18 Kohlenstoffatomen, dem Ethenylrest oder dem Ethinylrest, den Halogenatomen, den Gruppen $CF_3$, $SCF_3$, $OCF_3$, $NO_2$, $NH_2$ oder C≡N, dem Phenylrest, den Acyl- und Alkoxycarbonylgruppen mit 2 bis 8 Kohlenstoffatomen und den Alkylsulfonylgruppen mit 1 bis 6 Kohlenstoffatomen.

2. Verbindungen der Formel (I) gemäß Anspruch 1, worin R einen gegebenenfalls durch einen der in Patentanspruch 1 erwähnten Substituenten substituierten Phenylrest bedeutet.

3. Verbindungen der Formel (I) gemäß Anspruch 1 oder 2, worin R' für einen linearen, verzweigten oder cyclischen Alkylrest mit bis zu 12 Kohlenstoffatomen steht.

4. Verbindungen gemäß Anspruch 3, worin R' einen n-Pentyl-, n-Hexyl-, n-Heptyl oder n-Octylrest bedeutet.

5. Verbindungen der Formel (I) gemäß Anspruch 1 mit den folgenden Bezeichnungen

— 1-Benzoyl-5-(n-pentyloxy)-pyrrolidin-2-on

— 1-Benzoyl-5-(n-heptyloxy)-pyrrolidin-2-on.

6. Als Arzneimittel die Verbindungen gemäß einem der Ansprüche 1 bis 4.

7. Als Arzneimittel die Verbindungen gemäß Anspruch 5.

8. Pharmazeutische Zusammensetzungen, enthaltend als Wirkstoff zumindest ein Arzneimittel gemäß Anspruch 6 oder 7.

9. Verfahren zur Herstellung der Verbindungen der Formel (I) gemäß einem der Ansprüche 1 bis 5, dadurch gekennzeichnet, daß man eine Verbindung der Formel (II)

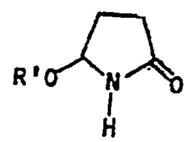

(II)

worin R′ die vorstehend angegebene Bedeutung besitzt, der Einwirkung einer Verbindung der Formel (III)

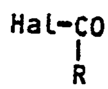

(III)

unterzieht, worin Hal ein Chlor- oder Bromatom bedeutet und R die vorstehend angegebene Bedeutung besitzt, um die entsprechende Verbindung der Formel (I) zu erhalten.

**Patentansprüche für folgenden vertragsstaat : ES**

1. Verfahren zur Herstellung der Verbindungen der Formel (I)

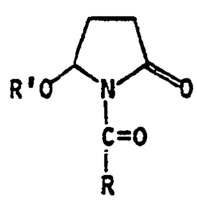

(I)

worin R′ für ein Wasserstoffatom, einen linearen, verzweigten oder cyclischen Alkylrest mit bis zu 12 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl oder Biphenylylrest oder einen Furyl-, Thienyl-, Pyranyl-, Pyridyl-, Benzofuranyl-Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno-[2,3-b]-furanyl-, 2H-Furo-[3,2b]-pyranyl-, Benzoxazolyl- oder Morpholinylrest bedeutet, wobei der Rest R gegebenenfalls substituiert sein kann durch einen oder mehrere Substituenten, ausgewählt unter dem Hydroxyrest, dem Acetoxyrest, dem Methoxyrest oder dem Benzyloxyrest, den gesättigten oder ungesättigten linearen, verzweigten oder cyclischen Alkylresten mit bis zu 18 Kohlenstoffatomen, dem Ethenylrest oder dem Ethinylrest, den Halogenatomen, den Gruppen $CF_3$, $SCF_3$, $OCF_3$, $NO_2$, $NH_2$ oder $C\equiv N$, dem Phenylrest, den Acyl- und Alkoxycarbonylgruppen mit 2 bis 8 Kohlenstoffatomen und den Alkylsulfonylgruppen mit 1 bis 6 Kohlenstoffatomen, dadurch gekennzeichnet, daß man eine Verbindung der Formel (II)

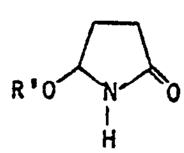

(II)

worin R′ die vorstehend angegebene Bedeutung besitzt, der Einwirkung einer Verbindung der Formel (III)

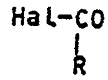

(III)

unterzieht, worin Hal ein Chlor- oder Bromatom bedeutet und R die vorstehend angegebene Bedeutung besitzt, um die entsprechende Verbindung der Formel (I) zu erhalten.

2. Verfahren gemäß Anspruch 1, dadurch gekennzeichnet, daß man von einer Verbindung der Formel (III) ausgeht, worin R einen gegebenenfalls durch einen der in Patentanspruch 1 erwähnten Substituenten substituierten Phenylrest bedeutet.

3. Verfahren gemäß Anspruch 1 oder 2, dadurch gekennzeichnet, daß man von einer Verbindung der Formel (II) ausgeht, worin R′ einen linearen, verzweigten oder cyclischen Alkylrest mit bis zu 12 Kohlenstoffatomen bedeutet.

4. Verfahren gemäß Anspruch 3, dadurch gekennzeichnet, daß man von einer Verbindung der Formel (II) ausgeht, worin R′ einen n-Pentyl-, n-Hexyl-, n-Heptyl oder n-Octylrest bedeutet.

5. Verfahren gemäß einem der Ansprüche 1 bis 4, dadurch gekennzeichnet, daß man von einer Verbindung der Formel (II), worin R′ für einen n-Pentyl- oder n-Heptylrest steht, und von einer Verbindung der Formel (III), worin R für einen Phenylrest steht, ausgeht.

**Patentansprüche für folgenden vertragsstaat : GR**

1. Verfahren zur Herstellung der Verbindungen der Formel (I)

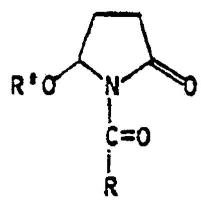

(I)

worin R′ für ein Wasserstoffatom, einen linearen, verzweigten oder cyclischen Alkylrest mit bis zu 12 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest steht und R einen Phenyl oder Biphenylylrest oder einen Furyl-, Thienyl-, Pyranyl-, Pyridyl-, Benzofuranyl-Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno-[2,3-b]-furanyl-, 2H-Furo-[3,2b]-pyranyl-, Benzoxazolyl- oder Morpholinylrest bedeutet, wobei der Rest R gegebenenfalls substituiert sein kann durch einen oder mehrere Substituenten, ausgewählt unter dem Hydroxyrest, dem Acetoxyrest, dem Methoxyrest oder dem Benzyloxyrest, den gesättigten oder ungesättigten linearen, verzweigten oder cyclischen Alkylresten mit bis zu 18 Kohlenstoffatomen, dem Ethenylrest oder dem Ethinylrest, den Halogenatomen, den Gruppen $CF_3$, $SCF_3$, $OCF_3$, $NO_2$, $NH_2$ oder C≡N, dem Phenylrest, den Acyl- und Alkoxycarbonylgruppen mit 2 bis 8 Kohlenstoffatomen und den Alkylsulfonylgruppen mit 1 bis 6 Kohlenstoffatomen, dadurch gekennzeichnet, daß man eine Verbindung der Formel (II)

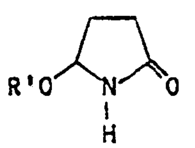

(II)

worin R′ die vorstehend angegebene Bedeutung besitzt, der Einwirkung einer Verbindung der Formel (III)

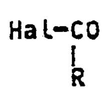

(III)

unterzieht, worin Hal ein Chlor- oder Bromatom bedeutet und R die vorstehend angegebene Bedeutung besitzt, um die entsprechende Verbindung der Formel (I) zu erhalten.

2. Verfahren gemäß Anspruch 1, dadurch gekennzeichnet, daß man von einer Verbindung der Formel (III)

ausgeht, worin R einen gegebenenfalls durch einen der in Patentanspruch 1 erwähnten Substituenten substituierten Phenylrest bedeutet.

3. Verfahren gemäß Anspruch 1 oder 2, dadurch gekennzeichnet, daß man von einer Verbindung der Formel (II) ausgeht, worin R' einen linearen, verzweigten oder cyclischen Alkylrest mit bis zu 12 Kohlenstoffatomen bedeutet.

4. Verfahren gemäß Anspruch 3, dadurch gekennzeichnet, daß man von einer Verbindung der Formel (II) ausgeht, worin R' einen n-Pentyl-, n-Hexyl-, n-Heptyl oder n-Octylrest bedeutet.

5. Verfahren gemäß einem der Ansprüche 1 bis 4, dadurch gekennzeichnet, daß man von einer Verbindung der Formel (II), worin R' für einen n-Pentyl- oder n-Heptylrest steht, und von einer Verbindung der Formel (III), worin R für einen Phenylrest steht, ausgeht.

6. Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, in eine für diese Verwendung vorgesehene Form bringt.

7. Verfahren zur Herstellung von pharmazeutischen Zusammensetzungen, dadurch gekennzeichnet, daß man als Wirkstoff zumindest eine der Verbindungen der Formel (I), wie in einem der Ansprüche 2 bis 4 definiert, in eine für diese Verwendung vorgesehene Form bringt.

8. Verfahren zur Herstellung von pharmazeutischen Zusammensetzungen, dadurch gekennzeichnet, daß man als Wirkstoff 1-Benzoyl-5-(n-pentyloxy)-pyrrolidin-2-on oder 1-Benzyl-5-(n-heptyloxy)-pyrrolidin-2-on in eine für diese Verwendung vorgesehene : Form bringt.

## Claims

### Claims for the following contracting States : AT, BE, CH, DE, FR, GB, IT, LI, LU, NL, SE

1. The compounds of formula (I) :

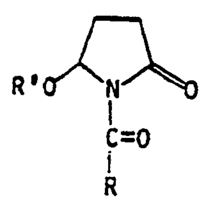

(I)

in which R' represents a hydrogen atom, a linear or branched alkyl radical containing up to 12 carbon atoms, an alkenyl radical containing 2 to 8 carbon atoms, an acyl radical containing 1 to 6 carbon atoms or a benzyl or phenethyl radical and R represents a phenyl or biphenylyl radical or one of the following radicals : furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2b]-pyranyl, benzoxazolyl or morpholinyl, the R radical being able to be optionally substituted by one or more substituents chosen from the group constituted by hydroxy, acetoxy, methoxy or benzyloxy radicals, saturated or unsaturated, linear, branched or cyclic alkyl radicals containing up to 18 carbon atoms, ethenyl or ethynyl radicals, halogen atoms, $CF_3$, $SCF_3$, $OCF_3$, $NO_2$, $NH_2$ or C≡N groups, the phenyl radical, acyl and alkoxy-carbonyl groups containing 2 to 8 carbon atoms and alkylsulphonyl groups containing 1 to 6 carbon atoms.

2. The compounds of formula (I) as defined in claim 1, in which R represents a phenyl radical optionally substituted by one of the substituents mentioned in claim 1.

3. The compounds of formula (I) as defined in claim 1 or 2, in which R' represents a linear, branched or cyclic alkyl radical containing up to 12 carbon atoms.

4. The compounds as defined in claim 3, in which R' represents an n-pentyl, n-hexyl, n-heptyl or n-octyl radical.

5. The compounds of formula (I) as defined in claim 1, the names of which follow :

— 1-benzoyl-5-(n-pentyloxy)-pyrrolidine-2-one,

— 1-benzoyl-5-(n-heptyloxy)-pyrrolidine-2-one.

6. As medicaments, the compounds as defined in any one of claims 1 to 4.

7. As medicaments, the compounds defined in claim 5.

8. The pharmaceutical compositions containing as active ingredient at least one medicament defined in claim 6 or 7.

9. Preparation process for the compounds of formula (I) as defined in any one of claims 1 to 5, characterized

in that a compound of formula (II) :

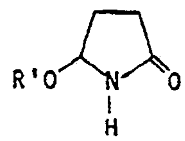

(II)

in which R' retains the same meaning as previously, is subjected to the action of a compound of formula (III) :

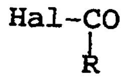

(III)

in which Hal represents a chlorine or bromine atom and R retains the same meaning as previously, in order to obtain the corresponding compound of formula (I).

**Claims for the following contracting States : ES**

1. Preparation process for compounds of formula (I) :

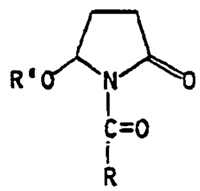

(I)

in which R' represents a hydrogen atom, a linear or branched alkyl radical containing up to 12 carbon atoms, an alkenyl radical containing 2 to 8 carbon atoms, an acyl radical containing 1 to 6 carbon atoms or a benzyl or phenethyl radical and R represents a phenyl or biphenylyl radical or one of the following radicals : furyl, thienyl, pyranyl, pyridyl, benzofuranyl, isobenzofuranyl, chromanyl, isochromanyl, chromenyl, xanthenyl, phenoxathienyl, oxazolyl, isooxazolyl, furazanyl, phenoxazinyl, thieno-[2,3-b]-furanyl, 2H-furo-[3,2b]-pyranyl, benzoxazolyl or morpholinyl, the R radical being able to be optionally substituted by one or more substituents chosen from the group constituted by hydroxy, acetoxy, methoxy or benzyloxy radicals, saturated or unsaturated, linear, branched or cyclic alkyl radicals containing up to 18 carbon atoms, ethenyl or ethynyl radicals, halogen atoms, $CF_3$, $SCF_3$, $OCF_3$, $NO_2$, $NH_2$ or C≡N groups, the phenyl radical, acyl and alkoxy-carbonyl groups containing 2 to 8 carbon atoms and alkylsulphonyl groups containing 1 to 6 carbon atoms, characterized in that a compound of formula (II) :

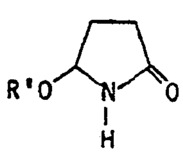

(II)

in which R' retains the same meaning as previously, is subjected to the action of a compound of formula (III) :

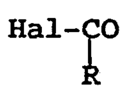

(III)

in which Hal represents a chlorine or bromine atom and R retains the same meaning as previously, in order to obtain the corresponding compound of formula (I).

2. Process according to claim 1, characterized in that a compound of formula (III) is used at the start in which R represents a phenyl radical optionally substituted by one of the substituents indicated in claim 1.

3. Process according to claim 1 or 2, characterized in that a compound of formula (II) is used at the start in which R' represents a linear, branched or cyclic alkyl radical containing up to 12 carbon atoms.

4. Process according to claim 3, characterized in that a compound of formula (II) is used at the start in which R′ represents an n-pentyl, n-hexyl, n-heptyl or n-octyl radical.

5. Process according to any one of claims 1 to 4, characterized in that a compound of formula (II) in which R′ represents an n-pentyl or n-heptyl radical and a compound of formula (III) in which R represents a phenyl radical are used at the start.

**Claims for the following contracting State : GR**

1. Preparation process for compounds of formula (I) :

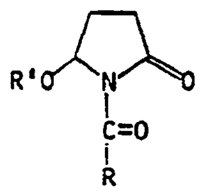

(I)

in which R′ represents a hydrogen atom, a linear or branched alkyl radical containing up to 12 carbon atoms, an alkenyl radical containing 2 to 8 carbon atoms, an acyl radical containing 1 to 6 carbon atoms or a benzyl or phenethyl radical and R represents a phenyl or biphenylyl radical or one of the following radicals : furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2b]-pyranyl, benzoxazolyl or morpholinyl, the R radical being able to be optionally substituted by one or more substituent chosen from the group constituted by hydroxy, acetoxy, methoxy or benzyloxy radicals, saturated or unsaturated, linear, branched or cyclic alkyl radicals containing up to 18 carbon atoms, ethenyl or ethynyl radicals, halogen atoms, $CF_3$, $SCF_3$, $OCF_3$, $NO_2$, $NH_2$ or C≡N groups, the phenyl radicals, acyl and alkoxy-carbonyl groups containing 2 to 8 carbon atoms and alkylsulphonyl groups containing 1 to 6 carbon atoms, characterized in that a compound of formula (II) :

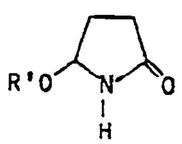

(II)

in which R′ retains the same meaning as previously, is subjected to the action of a compound of formula (III) :

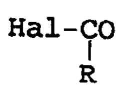

(III)

in which Hal represents a chlorine or bromine atom and R retains the same meaning as previously, in order to obtain the corresponding compound of formula (I).

2. Process according to claim 1, characterized in that a compound of formula (III) is used at the start in which R represents a phenyl radical optionally substituted by one of the substituents indicated in claim 1.

3. Process according to claim 1 or 2, characterized in that a compound of formula (II) is used at the start in which R′ represents a linear, branched or cyclic alkyl radical containing up to 12 carbon atoms.

4. Process according to claim 3, characterized in that a compound of formula (II) is used at the start in which R′ represents an n-pentyl, n-hexyl, n-heptyl or n-octyl radical.

5. Process according to any one of claims 1 to 4, characterized in that a compound of formula (II) in which R′ represents an n-pentyl or n-heptyl radical and a compound of formula (III) in which R represents a phenyl radical are used at the start.

6. Preparation process for pharmaceutical compositions, characterized in that at least one of the compounds of formula (I) as defined in claim 1, is used as active ingredient in a form intended for this use.

7. Preparation process for pharmaceutical compositions, characterized in that at least one of the compounds of formula (I) as defined in any one of claims 2 to 4, is used as active ingredient in a form intended for this use.

8. Preparation process for pharmaceutical compositions, characterized in that 1-benzoyl-5-(n-heptyloxy)-pyrrolidin-2-one is used as active ingredient in a form intended for this use.